# EUROPEAN PATENT SPECIFICATION

(11) **EP 1 945 175 B1**
(45) Date of publication and mention of the grant of the patent: **30.07.2014**
(21) Application number: 06827766.4
(22) Date of filing: 09.11.2006
(51) Int. Cl.: A61K 6/00

(54) **DENTURE ADHESIVE ARTICLE PACKAGING**
VERPACKUNG FÜR GEBISS-KLEBEARTIKEL
CONDITIONNEMENT D'ARTICLE ADHESIF POUR PROTHESE DENTAIRE

(30) Priority: 09.11.2005 US 735243 P; 09.11.2005 US 735088 P; 09.11.2005 US 735136 P; 09.11.2005 US 735135 P; 09.11.2005 US 734874 P; 20.01.2006 US 760528 P; 20.01.2006 US 760660 P; 20.01.2006 US 760516 P; 20.01.2006 US 760526 P; 20.01.2006 US 760527 P; 20.01.2006 US 760711 P; 31.10.2006 US 590145; 31.10.2006 US 590232; 31.10.2006 US 590224; 31.10.2006 US 590233; 31.10.2006 US 590111; 31.10.2006 US 590225; 31.10.2006 US 590191; 31.10.2006 US 590231
(43) Date of publication of application: 23.07.2008
(73) Proprietor: The Procter & Gamble Company, Cincinnati, OH 45202 (US)
(72) Inventor: RAJAIAH, Jayanth, Loveland, Ohio 45140 (US); MEDEIROS, Franco, Silva, Loveland, Ohio 45140 (US); WANG, Ping, Cincinnati, Ohio 45249-2406 (US); BOEHMER, Eric, Allen, Cincinnati, Ohio 45209-2020 (US)
(74) Representative: Töpert, Verena Clarita
(86) International application number: PCT/US2006/044030
(87) International publication number: WO 2007/056607

(56) References cited:
- WO-A-01/15657
- WO-A-01/41710
- WO-A-02/30317
- WO-A-96/25910
- WO-A-2005/081935

## Description

### FIELD OF THE INVENTION

The present invention relates to denture adhesive articles ready to use and package materials for such denture adhesive articles.

### BACKGROUND OF THE INVENTION

Ordinary removable dentures, dental plates and the like, comprise teeth mounted in a suitable plate or base. While dentures are traditionally fitted for the individual user, the fit can change over time which may result in slippage or discomfort. Denture adhesives are used to temporarily adhere the dentures to the surfaces of the oral cavity, in particular the oral mucosa. Denture adhesives are topically applied to either the denture or the oral surface at the beginning of the day when the dentures are placed into the oral cavity, and the adhesives tend to bio-erode during the course of the day due to action of saliva and chewing.

Considerable effort has been made over the years to develop improved denture adhesive products. Both synthetic and natural polymers and gums have been used alone and in combination with various adhesives and other materials in an attempt to improve hold and reduce oozing of the adhesive from under the dental plate, and to avoid messiness and difficulty of removing the residual adhesive from the mouth and dentures after use.

Recent developments in denture adhesives include denture adhesives provided as a strip or individually wrapped denture adhesive article. These compositions provide additional benefits over cream denture adhesives as they can be pre-measured into the correct amount to provide a particular level of adhesiveness and/or they can minimize perceived messiness when used by the consumer. However, due to the composition of these denture adhesive products, come of the articles can stick to the packaging materials particularly when exposed to elevated temperature and pressure, and can be difficult to remove from the packaging, especially by consumers with limited dexterity. Therefore, a need exists to provide a denture adhesive system which includes packaging from which the denture adhesive article can be easily removed and used by the consumer and which protects the denture adhesive article sufficiently to retain is composition and delivers the desired product benefit to the consumer.

### SUMMARY OF THE INVENTION

The present invention relates to a denture adhesive article delivery system comprising: a denture adhesive article ready to use, a primary package to enclose and retain the denture adhesive article, and a non-stick means for decreasing the sticking of a surface of the denture adhesive article to a surface of the primary package selected from the group consisting of a coating.

### BRIEF DESCRIPTION OF THE DRAWINGS

While the specification concludes with claims which particularly point out and distinctly claim the present invention, it is believed that the present invention will be better understood from the following description of preferred embodiments, taken in conjunction with the accompanying drawings, in which like reference numerals identify identical elements and wherein:
FIG. 1 is a perspective view of a denture adhesive article;
FIG. 2 is a cross-sectional view of a denture adhesive delivery system;
FIG. 3 is a cross-sectional view of a denture adhesive delivery system;
FIG. 4 is a side view of two denture adhesive articles within a liner;
FIG. 5 is a side view of two denture adhesive articles within a liner;
FIG. 6 is a cross-sectional view of a primary package comprising a non-stick coating;
FIG. 7 is a side view of a liner comprising a non-stick coating;
FIG. 8 is a perspective view of a denture adhesive article comprising a non-stick coating;
FIG. 9 is a cross-sectional view of a denture adhesive delivery system;
FIG. 10 is a perspective view of a denture adhesive article comprising a strip texture;
FIG. 11 is a perspective view of a denture adhesive article comprising a square texture;
FIG. 12 is a perspective view of a denture adhesive article comprising a triangle texture;
FIG. 13 is a perspective view of a denture adhesive article comprising a honeycomb texture;
FIG. 14 is a perspective view of a denture adhesive article comprising protrusions;
FIG. 15 is a side view of a liner comprising protrusions;
FIG. 16 is a cross-sectional view of a primary package comprising protrusions.

### DETAILED DESCRIPTION OF THE INVENTION

### Definitions

The term "denture" and/or "denature prosthesis" as used herein refers to either the upper or lower denture, or both.

The term "denture adhesive article" and/or "article" as used herein refers to articles designed to fit, conform and adhere to contoured surfaces, such as a denture, as well as the gums or the roof of the mouth. The articles herein are substantially solid prior to use and can be picked up manually in substantially one piece and positioned on the denture.

The term "denture adhesive delivery system" as used herein refers to the combination of a denture adhesive article, a primary package, and a means for decreasing the components of the denture adhesive delivery system from sticking and/or adhering to the other components of the system.

The term "denture adhesive article" as used herein refers to articles designed to fit, conform and adhere to contoured surfaces, such as a denture, as well as the gums or the roof of the mouth.

As used herein, the phrase "primary package" is intended to refer to the first packaging materials which enclose and/or retain and may be in contact with the denture adhesive article.

The term "flexible" or "flexible article" as used herein means that a 0.67 mm thick piece of the article may be wrapped 180 degrees around a solid cylinder of 1 cm diameter without cracking upon visual observation.

The term "safe and effective adhesive amounts" as used herein means an amount sufficient to provide adherence to the oral cavity and/or provide adherence of a denture to the oral cavity, without toxicity to the user or damage to oral tissue.

By "safe and effective amount", as used herein, is meant an amount of an agent high enough to significantly (positively) modify the condition to be treated or positively modify the benefit sought, but low enough to avoid serious side effects (at a reasonable benefit/risk ratio), within the scope of sound medical/dental judgment. The safe and effective amount of an agent may vary with the particular condition being treated, the age and physical condition of the patient being treated, the severity of the condition, the duration of treatment, the nature of concurrent therapy, the specific form of the source employed, and the particular vehicle from which the agent is applied.

The term "AVE/MA" as used herein refers to alkyl vinyl ether-maleic acid or anhydride copolymer. The term "mixed polymer salts" or "mixed salts", as used herein, refers to salts of AVE/MA where at least 2 different cations are mixed on the same polymer with each other or with other salts.

The term "free acid" or "FA" component, as used herein, refers either to the unreacted carboxyl groups (-COOH) of AVE/MA copolymer plus any other monovalent cations of carboxyl groups (e.g., COONa) of the polymer. Monovalent cations include Group IA cations, such as sodium, potassium, hydrogen, etc. In one embodiment, the term "free acid" refers to the unreacted carboxyl groups (-COOH) of AVE/MA plus sodium and potassium cations. In another embodiment, the term "free acid" refers only to the unreacted carboxyl groups (-COOH) of the AVE/MA.

The term "toxicologically-acceptable", as used herein, is used to describe materials that are suitable in their toxicity profile for administration to humans and/or animals.

By "non-aqueous", as used herein, is meant that the article does not contain added water but may contain water that is included in another component as supplied commercially by the manufacturer.

The term "water-insoluble" as used herein refers to a material that, when exposed to an excess of water, does not dissolve, but may disperse to varying degrees. In some embodiments the term "water-insoluble" refers to a material that is less than about 10%, 5%, 2%, or 1% soluble in water.

The term "thermoplastic" as used herein refers to a material that melts, softens, and becomes more flexible, extrudable, deformable, shapable, moldable, flowable, processable, and/or changes rheology when exposed to heat. In one embodiment the material generally solidifies, hardens, and/or substantially returns to its original condition, when subsequently cooled.

The term "bioerodible" as used herein means that the article, when exposed to excess of water or saliva, will erode over time due to physical and/or chemical action. The time necessary to erode the article can be any length of time from instantaneous to five days, in one embodiment the time to erode is from about 1 to about 3 days. The article may erode completely or substantially, however ultimately the article will lose its original form and/or integrity. For example, after application and use for at least about 24 hours in the oral cavity the article will not have sufficient product integrity to easily separate or peel, in its original form, from the denture or oral surface. In one embodiment the article bioerodes such that no portion of the article remains on the denture or mouth after the article has been used in the oral cavity for about 24 hours. In another embodiment some portion or residue from the article remains on the denture or oral surface after removing the denture from the oral cavity; however, this portion or residue from the article can be cleaned by brushing away with a toothbrush, but not easily separated from the denture.

The percentages used herein to describe the cationic salt function of the alkyl vinyl ether-maleic acid or anhydride copolymers are defined as the stoichiometric percent of the total initial carboxyl groups reacted on the polymer.

All other percentages used herein are by weight of the article unless otherwise indicated.

All measurements referred to herein are made at 25°C unless otherwise specified.

All percentages, ratios, and levels of ingredients referred to herein are based on the actual amount of the ingredient, and do not include solvents, fillers, or other materials with which the ingredient may be combined as a commercially available product, unless otherwise indicated.

### DENTURE ADHESIVE ARTICLE

The present invention relates to denture adhesive articles ready to use (e.g. shaped) designed to fit, conform and adhere to contoured surfaces such as a denture as well as the gums or the roof of the mouth.

In one embodiment the articles herein minimize or avoid the problem of premature sticking during application of the article to the denture. That is, with some prior art denture adhesive articles, before the article can be properly positioned over a targetsurface on the denture, inadvertent contact of the article with the denture may cause premature sticking at one or more locations on the denture. This may inhibit proper positioning of the article. Premature sticking may also cause contamination or degradation of the article prior to final positioning on the denture.

In one embodiment the term "dry tack" as used herein means that present articles exhibit minimal and/or no adhesive or cling properties in the dry state until activated by pressure applied by a user. In one embodiment this characteristic permits the present articles to be stored and dispensed in any desired mode without encountering the difficulties of premature clinging or adhering to themselves, and without the need for separate release sheets, liners, spacers, or the like. At the same time, in one embodiment when pressure activated at the desired location and at the desired time, the articles can, in the dry state, exhibit sufficient adhesive properties to form a bond to most plastic surfaces including a denture surface, this bond being sufficiently strong to survive handling of the denture without bond failure. Therefore, in one embodiment the articles herein, in the dry state, adhere to a target denture surface only when presses thereagainst, thereby minimizing or avoiding this problem of inadvertent adherence during positioning on the denture surface. In one embodiment then, the articles herein do not have to be moistened or wet prior to application to the denture, thus providing a simple and easy way to apply an article to the denture.

In one embodiment the term "dry tack" as used herein means that present articles exhibit minimal and/or no adhesive or cling properties until activated by pressure applied by the user after the article has been warmed by the hands of the user, potentially during the course of application of the article to the denture surface.

In another embodiment the articles herein are non-tacky to the touch prior to application to the denture.

In another embodiment the term "dry tack" as used herein means articles herein in a dry and un-wetted state, are capable of immediate bonding by surface attachment to a dry plastic, polymethyl methacrylate, and/or other denture prothesis substrate, upon subjecting the article to pressure. In one embodiment the dry article, develops bonding by surface attachment to a dry denture prosthesis substrate upon the application of finger pressure whereby the article remains bonded under its own weight, and the article herein will not remain bonded to this dry substrate under its own weight without using finger pressure to apply the article to the substrate. In one embodiment the force or pressure may be generated by one or more fingers. This force or finger pressure, in one embodiment, may be applied for 1-10 seconds or longer. In another embodiment the bonding of the article to the substrate is maintained from about 10 seconds to about 3 minutes or longer, in another embodiment from about 30 seconds to about 1 minute or longer.

In one embodiment the dry tack of the article is from about 0.025, 0.1, 1, 10, 100, 1000 gram force/square centimeter to about 10, 100, 1000, 10,000, 50,000, 100,000 gram force/square centimeter and any combination thereof.

In one embodiment the dry tack of an article that can be repositioned is from about 0.025 grams/force square centimeter to about 0.30 grams/force square centimeter, and in another embodiment from about 0.025 gram force/square centimeter to about 0.25 gram force/square centimeter.

It is reported that the room temperature modulus of any tacky adhesive is *less* than 3 x 10⁶ dynes/cm² when measured at a frequency of 1 Hz. This finding is a criterion for tack and has been given the name "Dahlquist criterion for tack." (Adhesion and Adhesives Technology, by Alphonsus Pocius, 2nd Edition, 2002 Carl Hanser Verlag, Munich).

In one embodiment of the current invention, the article has a modulus greater than the 'Dahlquist criterion for tack' of about 3 x 10⁶ dynes/cm². In another embodiment, the article has a shear storage modulus G' (measured in dynes/cm² at a frequency of about 1Hz at about 25C) greater than about 5 x 10⁶; in another embodiment greater than about 1 x 10⁷; in another embodiment greater than about 5 x 10⁷ ; and in another embodiment greater than about 8 x 10⁷.

In one embodiment the article has a shear storage modulus G' (measured in dynes/cm² at a frequency of about 1Hz at about 25C) from about 1 x 10⁶, 3 x 10⁶, 5 x 10⁶, 1 x 10⁷, 5 x 10⁷, and 8 x 10⁷ to about 5x 10⁸, 5 x 10⁷, 1 x 10⁸, 5 x 10⁹, 1 x 10⁹, and 1 x 10¹⁰ and/or any combination thereof.

In one embodiment the article has a flexural stiffness of less than about 10 grams/cm, in another embodiment less that about 5 grams/cm, in another embodiment less that about 3 grams/cm, in another embodiment less than about 2 grams/cm and in yet another embodiment from about 0.1, 0.5, 1, to about 2, 3, 5, 10 grams/cm, in any combination, flexural stiffness as measured on a Handle-O-Meter, model #211-300, available from Thwing-Albert Instrument Company of Philadelphia, PA as per test method ASTM D2923-95.

In one embodiment the articles herein have a normalized dislodgement force of from about 1100 to about 12,000 grams per sq.cm, in another embodiment from about 1300 to about 10,000 grams per sq.cm, in another embodiment from about 1200 to about 5000 grams per sq.cm, in another embodiment from about 1400 to about 5000 grams per sq.cm, in another embodiment from about 1300 to about 2500 grams per sq.cm, in another embodiment from about 1750 to about 2500 grams per sq.com. In another embodiment the normalized dislodgement force is from about 1100, about 1200, about 1300, about 1400, about 1500, about 1750 grams per sq.cm. to about 12,000, about 10,000, about 7500, about 5000, about 2500, about 2250 grams per sq.cm, and/or any combination thereof. In one embodiment the dislodgement force ratio is from about 1.1, 1.2, 1.3, 1.4, 1.5, 1.6, 1.7, 1.8, 1.9, 2.0 to about 1.3, 1.4, 1.5, 1.6, 1.7, 1.8, 1.9, 2.0, 3, 4, 5, 6, 8, 10, and/or any combination thereof. In one embodiment the dislodgement force ratio is from about 1.1 to about 10, from about 1.1 to about 8, from about 1.3 to about 4, and/or from about 1.3 to about 2.5.

The articles herein are substantially solid prior to use and can be picked up manually, in substantially one piece, and positioned on the denture. Additionally, in one embodiment the articles are capable of being picked up manually, and positioned on the denture, resulting in little or no residue on the fingers. In another embodiment the articles comprise a single layer. In yet another embodiment the articles are laminates, and/or composites. In one embodiment the articles are pre-shaped and/or preformed.

Regardless of the form of dispensing the article, including ready to use articles the articles are substantially solid prior to use and can be picked up manually.

Some denture adhesive articles are ready to use. A user may be able to identify these items visually as a denture adhesive article, as they are often in the form of a strip contained within a package. However, if not evident that these denture adhesive products are articles, these denture adhesive articles can be identified as articles by the following method:
1. Shape composition into a sheet about 0.67 mm thick x about 8 mm wide x about 44 mm long.
2. Place sheet on a denture-tile.
3. Use fingers to pick up sheet.
4. The composition is an article if it can be picked up in substantially one piece.

Substantially in one piece means, as used herein, that from about 75, 80, 85, 90% to about 100, 90, 85, 80, 75, 70% and/or any combination thereof of the denture adhesive composition remains in one piece when manually picked up from the denture surface.

In addition to the aforementioned test methods denture adhesive articles can also be identified as articles by the amount of ooze, as determined by the ooze method (as defined herein). In addition to being able to be manually picked up and moved in substantially one piece, a denture adhesive article has a normalized ooze amount of from about 0, 3, 5, 10, 15, 20, 25% of the total composition to about 30, 25, 20, 15, 10, 5, 3 % of the total composition and/or any combination thereof and/or the ooze ratio is from about 0, 00001, 0.001, 0.01, 0.1, 0.2, 0.25, 0.3, to about 0.1, 0.2, 0.25, 0.3, 0.4, 0.5 and/or any combination thereof.

In one embodiment the article herein comprises ingredients of natural origin.

In one embodiment the article herein comprises a homogeneous mixture of the denture adhesive component and the water insoluble thermo-plastic component.

The denture adhesive article can have a variety of shapes and sizes including but not limited to a concave shape which is either symmetrical or asymmetrical.

### DENTURE ADHESIVE COMPONENT

The present invention comprises a safe and effective adhesive amount of a denture adhesive component, generally at a level of from about 10% to about 90%, in another embodiment from about 15% to about 70%, in another embodiment from about 20% to about 70%, in yet and in another embodiment from about 25% to about 65%, and in yet another embodiment from about 30% to about 65%, by weight of the article. In one embodiment the articles of the present invention comprise from at least 20 percent by weight, and in another embodiment at least 30 percent by weight of the article, of a denture adhesive component.

In one embodiment the denture adhesive components herein are mucoadhesive, hydrophilic, water soluble, have the property of swelling upon exposure to moisture, and/or to form a mucilaginous mass when combined with moisture. In one embodiment the denture adhesive components are selected from the group consisting of natural gums, synthetic polymeric gums, AVE/MA, salts of AVE/MA, AVE/MA/IB, salts of AVE/MA/IB, copolymer of maleic acid or anhydride and ethylene and salts thereof, copolymer of maleic acid or anhydride and styrene and salts thereof, copolymer of maleic acid or anhydride and isobutylene and salts thereof, polyacrylic acid and polyacrylates thereof, polyitaconic acid and salts thereof, mucoadhesive polymers, water-soluble hydrophilic colloids, saccharide derivatives, cellulose derivatives, and mixtures thereof. Examples of such materials include karaya gum, guar gum, gelatin, algin, sodium alginate, tragacanth, chitosan, acrylamide polymers, carbopol, polyvinyl alcohol, polyamines, polyquarternary compounds, polyvinylpyrrolidone, cationic polyacrylamide polymers, AVE/MA, AVE/MA/IB, mixed salts of AVE/MA, mixed salts of AVE/MA/IB, polymeric acids, polymeric salts, polyhydroxy compounds, and mixtures thereof.

In one embodiment the denture adhesive components are selected from the group consisting of salts of AVE/MA, mixed salts of AVE/MA, cellulose derivatives (such as methylcellulose, carboxymethylcellulose, hydroxyethylcellulose, hydroxypropylcellulose, hydroxy-propylmethylcellulose, and mixtures thereof), polyethylene glycol, karaya gum, sodium alginate, chitosan, corn starch, and mixtures thereof. In yet another embodiment, the adhesive component is selected from the group consisting of mixed salts of AVE/MA, cellulose derivatives, and mixtures thereof.

In one embodiment the denture adhesive component is not thermoplastic and/or comprises only low levels of water soluble thermoplastic polymers, from about 0.01 to about 5% of water soluble thermoplastic polymer such as polyethylene oxide, hydroxypropyl cellulose, hydroxyproplymethylcellulose; polyethylene glycol; in another embodiment from about 0.01 to about 1% of water soluble thermoplastic polymer, or is essentially free of water soluble thermoplastic polymers.

### Alkyl Vinyl Ether-Maleic Copolymer

In one embodiment of the invention the denture adhesive component is AVE/MA or salts of AVE/MA. The alkyl vinyl ether-maleic acid co-polymer comprises or consists essentially of the repeated structural unit: 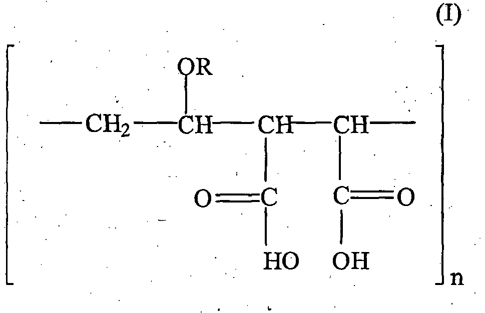 wherein R represents an alkyl radical, in one embodiment a C₁ to C₅ alkyl radical, n is an integer greater than one representing the number of repeated occurrences of the structural unit in a molecule of the polymer.

In one embodiment, the adhesive component is AVE/MA and salts thereof, preferably mixed salts of AVE/MA, wherein the copolymer contains a cationic salt function comprising a cation selected from the group consisting of Group IA and Group 2A cations of the periodic table, yttrium, titanium, zirconium, vanadium, chromium, manganese, iron, nickel, copper, zinc, boron, aluminum, and mixtures thereof. In another embodiment, the adhesive component is a mixed salt of AVE/MA containing a cationic salt function comprising a cation selected from the group consisting of strontium, zinc, iron, boron, aluminum, vanadium, chromium, manganese, nickel, copper, yttrium, titanium, magnesium, calcium, sodium, and mixtures thereof, and in yet another embodiment the cation is selected from the group consisting of strontium, zinc, iron, magnesium, calcium, sodium, and mixtures thereof.

AVE/MA contains, in one embodiment, a cationic salt function comprising from about 5% to about 50%, in another embodiment, from about 10% to about 40%, in yet another embodiment, from about 10% to about 35% (of the total initial carboxyl groups reacted) zinc cations. These zinc cations can be mixed with other cations selected from the group consisting of: from about 5% to about 65%, preferably from about 10% to about 60%, strontium cations, from about 0.001% to about 2.5%, preferably from about 0.01% to about 2% of iron, boron, aluminum, vanadium, chromium, manganese, nickel, copper, yttrium; and/or titanium cations, from about 5% to about 65%, preferably from about 15% to about 50% of calcium and/or magnesium cations and/or sodium cations.

AVE/MA and salts thereof, are also described in U.S. Patent Nos. 5,073,604 to Holeva et al., issued 12/17/91; 5,525,652, issued Jun. 11, 1996, Clarke et al.; 6,025,411, issued Feb. 15, 2000, Wong et. al.; 4,758,630, issued July 19, 1988, Shah et al.; 5,304, 616, issued April 19, 1994, Rajaiah et al.; 5,424,058, issued June 13, 1995, Rajaiah; 5,424,058, issued 6/13/95, Rajaiah et al.; 4,758,630, issued July 19, 1988, Shah et al.; 5,830,933, issued Nov. 3, 1998, Synodis et al.; 2,047,398, issued July 14, 1936, Voss et al.; 3,003,988, issued Oct. 10, 1961, Germann et al.; 5,880,172, Rajaiah et al., issued March 9, 1999; 5,900,470, Prosise et al., issued 5/4/99; 5,037,924, Tazi et al., issued 8/6/91; 5,082,913, Tazi et al, issued 1/21/92. Salts of AVE/MA are also described in P&G copending applications U.S. Patent Nos. 6,355,706 to Rajaiah, et al., issued March 12, 2002; 6,617,374 to Rajaiah, et al., issued September 9, 2003.

In one embodiment the free acid level of the salts of the AVE/MA or AVE/MA/IB is at least about 36%, in another embodiment is from about 36% to about 60%, and even in another embodiment is from about 40% to about 55%, of the total initial carboxyl groups of the copolymer.

In one embodiment the specific viscosity of the starting copolymer acid or copolymer anhydride is from about 1.2 to about 14, when preferably measured in a 1% weight/volume solution in MEK (methyl ethyl ketone) at 25°C. Other methods and solvents can be used to measure the specific viscosity such as a 1% weight/volume solution in DMF (dimethyl formamide) at 25°C and a 1% weight/volume solution in 2-butanone at 25°C.

Suitable AVE/MA copolymers may be prepared by well-known methods of the prior art; see, for example, US 2,782,182, and US 2,047,398.

Methods of making mixed salts of AVE/MA polymers are further disclosed in U.S. Patent Nos. 5,073,604, Holeva et al., issued Dec. 17, 1991 and 5,872,161, Liang et al., issued Feb. 16, 1999.

### WATER INSOLUBLE COMPONENT

The present article comprises a safe and effective amount of a water insoluble component. In one embodiment this component is at a level from about 2, 5, 10, 20, 25, 30, 35% to about 45, 50, 60, 70, 90%, and/or any combination thereof to create ranges, by weight of the article, in another embodiment the water insoluble component level is from about 20% to about 70%, from about 25% to about 60%, or from about 35% to about 60% by weight of the article. In yet another embodiment the water insoluble component is both water insoluble and substantially non-swellable in water.

The water insoluble component is a water insoluble thermoplastic component that is selected from the group consisting of rubber, elastomers, plastomers, natural wax, synthetic wax, polyvinyl chloride, nylon, fluorocarbon, polyurethane prepolymer, polyethylene, polystyrene, polypropylene, cellulosic resins, acrylic resins, petrolatum, poly vinyl acetate and mixtures thereof. In another embodiment the water insoluble thermoplastic component is selected from the group consisting of natural wax, synthetic wax, petrolatum, polyethylene, and mixtures thereof. In yet another embodiment the water insoluble thermoplastic component is selected from the group consisting of polyethylene, petrolatum, paraffin wax, microcrystalline wax, polypropylene, polystyrene, and mixtures thereof; in another embodiment is selected from the group consisting of polyethylene, microcrystalline wax, and mixtures thereof.

In one embodiment the water insoluble thermoplastic component comprises elastomers such as Ethylene-Ethylene-Propylene rubber, Ethylene-Propylene rubber, Styrene-Ethylene-Ethylene-Ethylene-Propylene-Styrene rubber, and combinations thereof; and these may optionally be further combined with waxes.

In one embodiment the article herein has a water insoluble thermoplastic component having a penetration of about 25 to about 350; in another embodiment from about 27 to about 250 in another embodiment from about 30 to about 150, and in another embodiment from about 40 to about 150, and in another embodiment from about 50 to about 80, and in yet another embodiment from about 60 to about 80. The penetration values are from about 25 to about 250 when measured using the ASTM D 1321 method and the penetration values are from about 25 to about 350 when measured using the ASTM D937 method, both are existing methods known in the art. Although ASTM D937 and ASTM D1321 generally are used to measure the penetration of petrolatum or petroleum waxes, respectively, these methods may be used to measure the penetration of waxes derived from petroleum and other types of water insoluble thermoplastic components with appropriate modifications, for example, these other components may need to be melted at higher temperature which will be apparent to one of skill in the art.

In obtaining the above penetration values, water insoluble thermoplastic components that are outside of these penetration values may be mixes with another ingredient that modifies the penetration of the thermoplastic component. Therefore the water insoluble thermoplastic component may be a single ingredient or may be a mixture of ingredients. For example, Multiwax W 180 manufactured by Witco (Crompton, Sonneborn), having a penetration value of about 15 to about 20 may be mixed with petrolatum (at a 1:1 ratio) to raise the penetration value to above 25.

In one embodiment the water insoluble thermoplastic component is a natural or synthetic wax. These waxes include natural waxes such as animal, vegetable, mineral wax. Animal waxes include beeswax, lanolin, shellac wax, Chinese wax, etc. Vegetable waxes include carnauba, candelilla, bayberry, sugar cane, etc., and mineral waxes include fossil or earth waxes (ozocerite, ceresin, montan), and petroleum waxes such as paraffin, microcrystalline, etc. In one embodiment the waxes herein are natural waxes selected from the group consisting of beeswax, candelilla, candela, carnauba, paraffin, microcrystalline wax, Fischer-Tropsch waxes, and mixtures thereof.

In another embodiment the wax is microcrystalline wax manufactured by Crompton, Sonneborn (Witco) and referred to and sold under the trademark MutiwaxW-835. This wax has a melt point ranging from about 73.9 °C to about 79.4 °C (ASTM D127), has a penetration at 25° of from about 60 to about 80 (ASTM D1321), has a kinematic viscosity at 98.9° C, of from about 75 to about 90 (ASTM D2161), has a flash point, COC, of about 246 °C Min. (ASTM D92), and has a congealing point, of about 77 °C (ASTM D938).

In one embodiment the paraffin waxes useful herein generally can have a melting point range of from about 65° C to about 80° C, in another embodiment about 70° C to about 75° C; the microcrystalline wax useful herein can have a melting point of from about 65° C to about 90° C, in another embodiment can have a melting point of from about 80° C to about 90° C; the beeswax useful herein can have a melting point of from about 62° to about 65° C with a flash point of 242° C; the candelilla wax useful herein can have a melting point of from about 68° to about 72° C; the carnauba wax useful herein can have a melting point of from about 83° to about 86° C; the Fischer-Tropsch wax useful herein can have a melting point of about 95° to about 120° C. Synthetic grades of beeswax, candelilla, and carnauba waxes are also available with similar properties as the natural grades.

In another embodiment the water insoluble thermoplastic component is polyethylene such as A-C 1702 and A-C 6702 made by Honeywell, with a penetration value of 98.5 and 90.0, respectively, under ASTM D1321.

In one embodiment if the article contains polyethylene oxide, then either the water insoluble component is thermoplastic or the article may not include a fibrous paper web or paper laminate.

In one embodiment the article herein is substantially free of honey mixed with alcohol. In another embodiment the article is substantially free of polyvinyl acetate resin in ethyl alcohol.

### MISCELLANEOUS OPTIONAL INGREDIENTS

### Plasticizing Agent

The articles of the present invention may also optionally comprise a safe and effective amount of one or more toxicologically-acceptable plasticizers. In one embodiment the level of the plasticizing agent ranges from about 0.0% to about 40%, in one embodiment from about 0.01% to about 40%, in another embodiment from about 1% to about 10%, in another embodiment from about 2% to about 5%, by weight of the article. In yet another embodiment the denture adhesive article does not comprise a plasticizer.

Suitable plasticizing agents of the present invention include, but are not limited to, polyols (such as sorbitol); glycerin; propylene glycol; acetylated monoglyceride; hydrogenated starch hydrolysates; corn syrups; and derivatives thereof; xylitol, glycerol monoesters with fatty acids; triacetin; diacetin; and monoacetin; dimethyl phthalate; diethyl phthalate; dioctyl phthalate; diethylene glycol; triethylene glycol; tricresyl phosphate; dimethyl sebacate; ethyl glycolate; ethylphthalyl ethyl glycolate; o- and p-toluene ethyl sulfonamide; phthalic acid derivative, glycerol triacetate, citric acid derivative, phosphoric acid derivative, glycol, glycol derivative, paraffin wax, a pentaerythritol ester of a fatty acid, stearic acid derivative, glycerol monostearate, polyethylene glycol, butyl phthalyl butyl glycolate, butyl phthalyl butyl glycolate, dimethyl phthalate, dibutyl phthalate, triacetin, triethyl citrate, acetyl triethyl citrate, acetyl tributyl citrate, triphenyl phosphate, diethylene glycol, caprylic triglyceride, capric triglyceride, propylene glycol dicaprylate/caprate and/or combinations thereof.

In another embodiment the plasticizer is water insoluble.

In one embodiment, the denture adhesive article, when extruded thermoplastically, does not cure and set as a result of the action of the plasticizer component. In another embodiment the plasticizer component does not solidify the water insoluble component or the denture adhesive article. In another embodiment the water insoluble thermoplastic component does not cure and set.

Alternatively, in one embodiment the denture adhesive article may be substantially free of plasticizers. In one embodiment the denture adhesive article can be substantially free of polyethylmethacrylate, triacetin, phthalic acid derivative, glycerol triacetate, citric acid derivative, phosphoric acid derivative, glycol, glycol derivative, paraffin wax, a pentaerythritol ester of a fatty acid, stearic acid derivative, glycerol monostearate, polyethylene glycol, butyl phthalyl butyl glycolate, butyl phthalyl butyl glycolate, dimethyl phthalate, dibutyl phthalate, triacetin, triethyl citrate, acetyl triethyl citrate, acetyl tributyl citrate, triphenyl phosphate, diethylene glycol, caprylic triglyceride, capric triglyceride, propylene glycol dicaprylate/caprate and/or combinations thereof.

### Gellant Agents.

The articles of the present invention may also optionally comprise a safe and effective amount of one or more toxicologically-acceptable gellants. In one embodiment the level of the gellant agent ranges from about 0.01% to about 40%, in another embodiment from about 1% to about 10%, in another embodiment from about 2% to about 5%, by weight of the article. Suitable gellant agents of the present invention include, but are not limited to, polyvinylpyrrolidone/eicosene copolymer sold under the trade name Ganex V-220F from ISP; tricontanyl polyvinylpyrrolidone sold under the trade name Ganex WP-660 from ISP; polyamide gallants including Sylvaclear, Sylvacote, Sylvagel, Uniclear all available from Arizona Chemical including Sylvaclear Lightwax; Sylvaclear PA 20; Sylvaclear PA 30; Sylvaclear PA 50; Sylvacote 2228; Sylvacote 2228E; Sylvagel 5000; Sylvagel 6000; Uniclear 100; Uniclear 100VG; Uniclear 80; Uniclear 80V; and mixtures thereof.

### Flavors, Fragrance, Sensates

The articles of the present invention may also include one or more components which provide flavor, fragrance, and/or sensate benefit (warming or cooling agents). Suitable components include menthol, wintergreen oil, peppermint oil, spearmint oil, leaf alcohol, clove bud oil, anethole, methyl salicylate, eucalyptol, cassia, 1-8 menthyl acetate, sage, eugenol, parsley oil, oxanone, alpha-irisone, marjoram, lemon, orange, propenyl guaethol, cinnamon, vanillin, thymol, linalool, cinnamaldehyde glycerol acetal known as CGA, and mixtures thereof, as well as coolants. The coolant can be any of a wide variety of materials. Included among such materials are carboxamides, menthol, ketals, diols, and mixtures thereof. In one embodiment the coolants in the present articles are selected from the group consisting of the paramenthan carboxyamide agents such as N-ethyl-p-menthan-3-carboxamide, known commercially as "WS-3", N,2,3-trimethyl-2-isopropylbutanamide, known as "WS-23," and mixtures thereof. Additional preferred coolants are selected from the group consisting of menthol, 3-1-menthoxypropane-1,2-diol known as TK-10 manufactured by Takasago, menthone glycerol acetal known as MGA manufactured by Haarmann and Reimer, and menthyl lactate known as Frescolat® manufactured by Haarmann and Reimer. The terms menthol and menthyl as used herein include dextro- and levorotatory isomers of these compounds and racemic mixtures thereof. TK-10 is described in U.S. Pat. No. 4,459,425, Amano et al., issued 7/10/84. WS-3 and other agents are described in U.S. Pat. No. 4,136,163, Watson, et al., issued Jan. 23, 1979. These agents may be present at a level of from about 0% to about 40%, in another embodiment from about 0.05 to about 5%, and in another embodiment from about 0.1 to about 2%, by weight of the article.

### Other Optional Ingredients

The denture adhesive articles may also comprise one or more therapeutic actives suitable for topical administration. Therapeutic actives may be present at a level of from about 0% to about 70%, by weight of the article, and in one embodiment from about 1% to about 20% by weight of the article. Therapeutic actives include antimicrobial agents such as iodine, triclosan, peroxides, sulfonamides, bisbiguanides, or phenolics; antibiotics such as tetracycline, neomycin, kanamycin, metronidazole, cetylpyridium chloride, domiphen bromide, or clindamycin; anti-inflammatory agents such as aspirin, acetaminophen, naproxen and its salts, ibuprofen, ketorolac, flurbiprofen, indomethacin, eugenol, or hydrocortisone; dentinal desensitizing agents such as potassium nitrate, strontium chloride or sodium fluoride; fluorides such as sodium fluoride, stannous fluoride, MFP; anesthetic agents such as lidocaine or benzocaine; anti-fungals such as those for the treatment of *candida albicans;* aromatics such as camphor, eucalyptus oil, and aldehyde derivatives such as benzaldehyde; insulin; steroids; herbal and other plant derived remedies; and baking soda. It is recognized that in certain forms of therapy, combinations of these agents in the same delivery system may be useful in order to obtain an optimal effect. Thus, for example, an antimicrobial and an anti-inflammatory agent may be combined in a single delivery system to provide combined effectiveness.

Other suitable ingredients include colorants, preservatives (such as methyl and propyl parabens), thickeners such as silicon dioxide, and polyethylene glycol. Colorants, preservatives, thickeners may be present at levels of from about 0% to about 20%, by weight of the article, in another embodiment from about 0.1% to about 10%, by weight.

Additionally, the articles may also comprise one or more solvents. These optional solvents may be miscible with the water insoluble component and/or be capable of being dissipated in-situ. In one embodiment these solvents may be dissipated in-situ by evaporation, dissolution, dispersion, bio-absorption, or any other suitable means. In another embodiment these solvents may be dissipated in-situ to leave behind a denture adhesive article. Such solvents may include materials with a viscosity ranging from 0.01, 0.1, 1, 5 centipoise 20°C, to 5, 10, 100, 1000 centipoise at 20°C in any combination of these levels. In one embodiment these solvents may be silicones, hydrocarbons, iso-dodecane, iso-hexadecane, iso-eicosane, and/or polyisobutene. Suitable grades of solvents include the Permethyl series (sold by Prespers Inc., New Jersey) such as Permethyl 97A, 99A, 101A, 102A, and mixtures thereof.

### PRIMARY PACKAGE

The primary package can comprise any material which can be in contact with the components of the denture adhesive delivery system, without negatively reacting with the other components of the denture adhesive delivery system, while enclosing and retaining the denture adhesive article(s). The primary packaging can be sealed about the peripheral edge. The primary package will retain the denture adhesive article until the user opens the primary package and removes the denture adhesive article for use and application to the denture and/or oral surface. In one embodiment the primary package comprises polymers including, but not limited to, polyethylene, ethylvinylacetate, ethylvinyl alcohol, polyesters such as Mylar manufactured by DuPont, fluoroplastics such as Teflon manufactured by DuPont, and combinations thereof. Additionally, in one embodiment the primary package can be formed from a polyolefin, for example from polyethylene or polypropylene. In another embodiment the primary package, can comprise polyolefin blends, polyethylene blends, polypropylene blends, and/or combinations thereof. In one embodiment the primary package can be a laminate of various materials including, foil, polyethylene, polypropylene, etc.

### NON-STICK PACKAGING

The aforementioned denture adhesive article may stick to packaging materials due to its composition, in particular if the denture adhesive article is exposed to extreme temperatures during storage and shipping. The denture adhesive article may be more likely to stick to the packaging if water insoluble thermoplastic component of the denture adhesive article comprises rubber, elastomers, plastomers, natural wax, synthetic wax, polyvinyl chloride, nylon, fluorocarbon, polyurethane prepolymer, polyethylene, polystyrene, polypropylene, cellulosic resins, acrylic resins, petrolatum, poly vinyl acetate and mixtures thereof. In another embodiment the water insoluble thermoplastic component is selected from the group consisting of natural wax, synthetic wax, petrolatum, polyethylene, and mixtures thereof. In yet another embodiment the water insoluble thermoplastic component is selected from the group consisting of polyethylene, petrolatum, paraffin wax, microcrystalline wax, polypropylene, polystyrene, and mixtures thereof; in another embodiment is selected from the group consisting of polyethylene, microcrystalline wax, and mixtures thereof. These aforementioned insoluble thermoplastic components may be more likely to change to either adhere to the package of the present invention, or portions of the components of the denture adhesive article may be absorbed by the packaging materials.

To minimize the sticking of the denture adhesive article to the packaging materials, a variety of means can be used, including, but not limited to: (i) placing a liner between the primary package and the denture adhesive article, (ii) coating the primary package, the denture adhesive article and/or the liner with a non-stick composition such as silicone; and (iii) minimizing the contact area between the denture adhesive article and the primary package and/or the liner, and (iv) any combination of any of these means. These means can also

### Liner

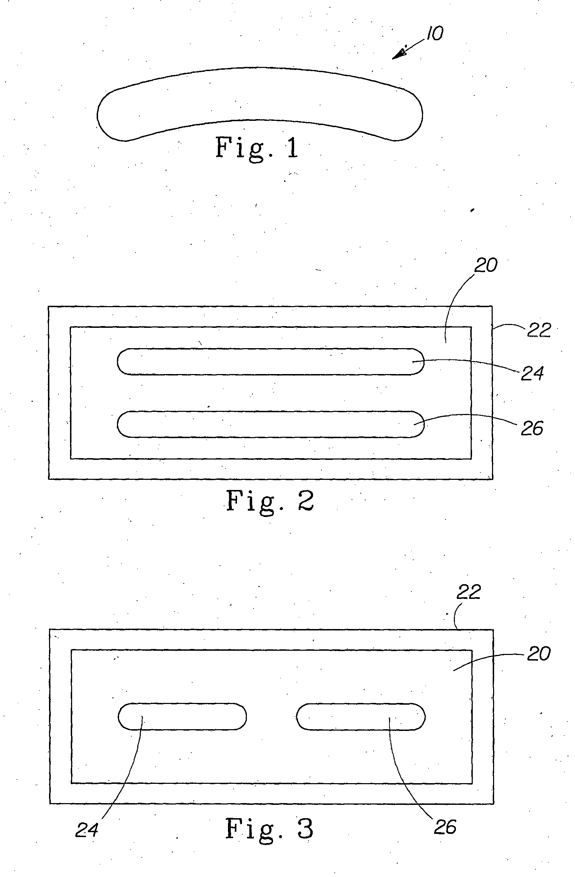

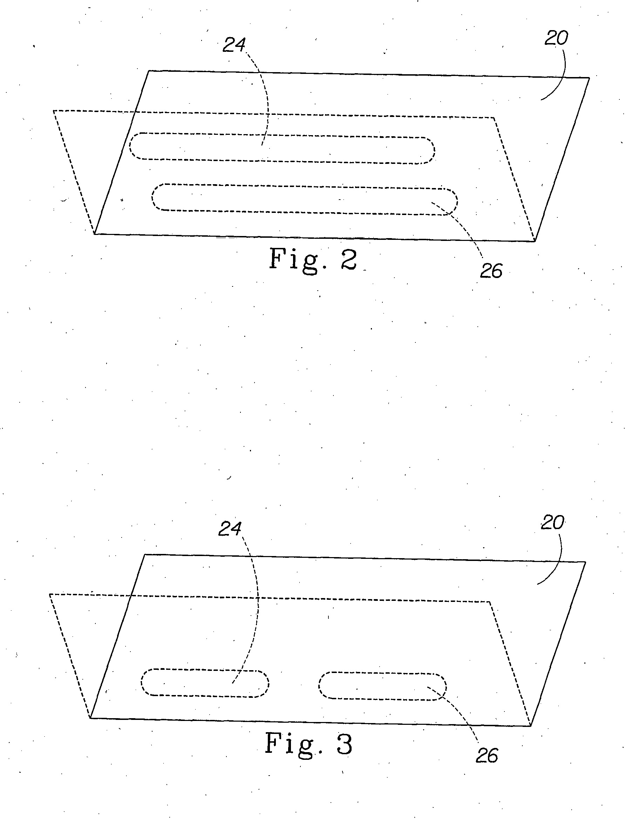

In one example not comprised by the invention sticking of the denture adhesive article to the primary package can be minimized by including a liner 20 between the primary package 22 and the denture adhesive articles 24 and 26 (as shown in Figs. 2-5). The denture adhesive articles 24 and 26 can be placed between two separate liners (not shown) or a single liner 20 folded to contact both sides of the denture adhesive article (as shown in Fig. 4-5). The denture adhesive articles can be disposed either stacked side to side (as shown in Figs. 2 and 4) or end to end (as shown in Figs. 3 and 5). The liner 20 can be any material for which the denture adhesive article has little or no affinity, including but not limited to, parchment paper, silicone rubber, silicone foams, silicone coated films, silicone coated paper, silicone-impregnated paper, hydro-formed films, textured films, and/or laminates. In one embodiment the liner is parchment paper. In another embodiment the liner is parchment paper impregnated with silicone. The liner can be substantially smooth, or it can be textured.

### Coating

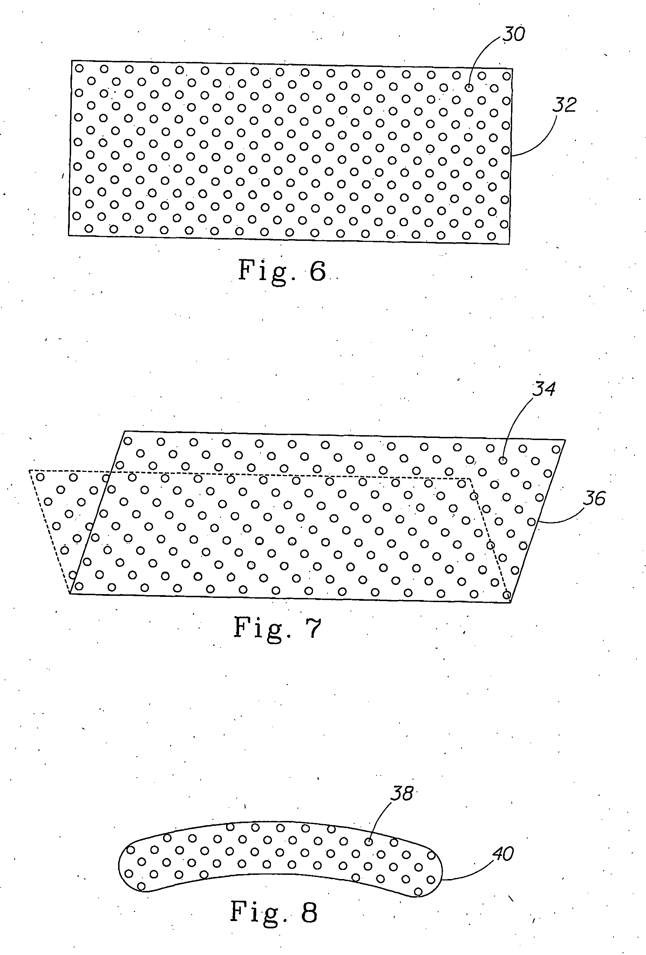

The sticking of the denture adhesive article to the primary package is minimized by coating the denture adhesive article, the primary package, and/or the liner (as shown in Figs. 6-8). The coating can include silicone compounds, silicone polymers, heat cured silicones, crosslinked silicones, fluoro compounds, and/or UV cured silicones. The coating can be applied prior to packaging the denture adhesive article within the primary package. The coating 30 can be applied to the interior surface of the primary packaging 32, the coating 34 can be applied to the contact surfaces of the liner 36, and/or the coating 38 can be applied to the contact surfaces of the article 40. The coating can be applied to any or all of the surfaces of the denture adhesive delivery system, including but not limited to, all the surfaces that are contact surfaces. This coating can be applied onto the primary package, adhesive and/or liner using any suitable method including spray-coating, roll-coating, slot-coating, printing, and/or brush-coating.

### Minimizing Surface Contact

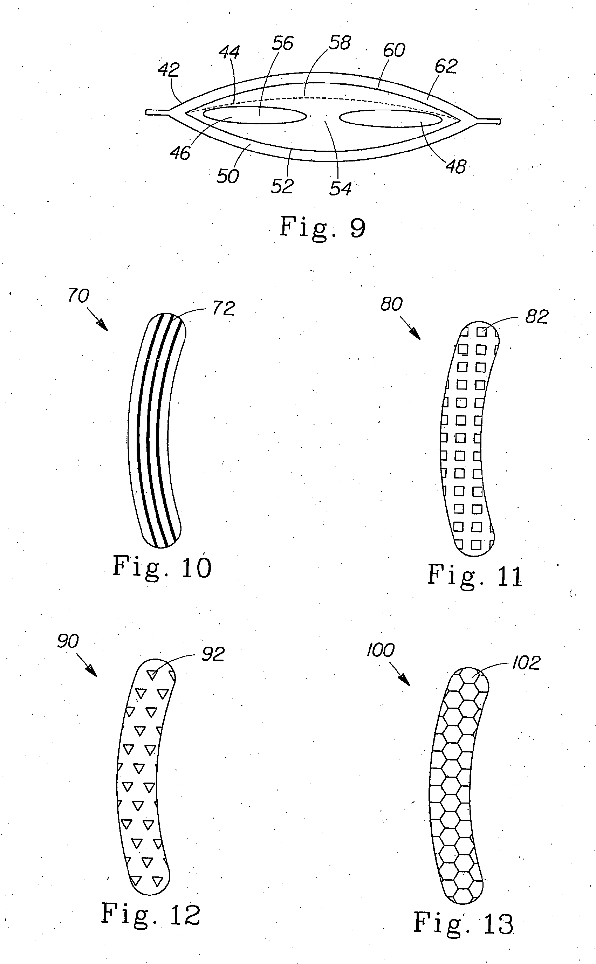

In yet another embodiment the contact area between the denture adhesive article and the primary package and/or the liner can be minimized by changing the contact surface of the article, the primary package and/or the liner. As the touching portion of the surface area of the adjacent contact surfaces increases, so does the likelihood that the adjacent contact surfaces will adhere to each other. The contact surface is a surface of the article, primary package, and/or liner which comes into contact with another of the surfaces of the article, primary package, and/or the liner. For example (as shown in Fig. 9) the primary package 42 contact surface 50 touches the exterior contact surface 52 of the liner 44; and the interior contact surface 54 of the liner 44 touches the contact surface (not shown) of the articles 46 and 48, and the contact surface 56 of the article 46 touches the contact surface 58 of the liner 44; and the outside contact surface 60 of the liner 44 touches the contact surface 62 of the primary package 42. All of surfaces denture adhesive article 46 and 48 are contact surfaces; as all the surfaces contact either the liner 44 or the primary package 42.

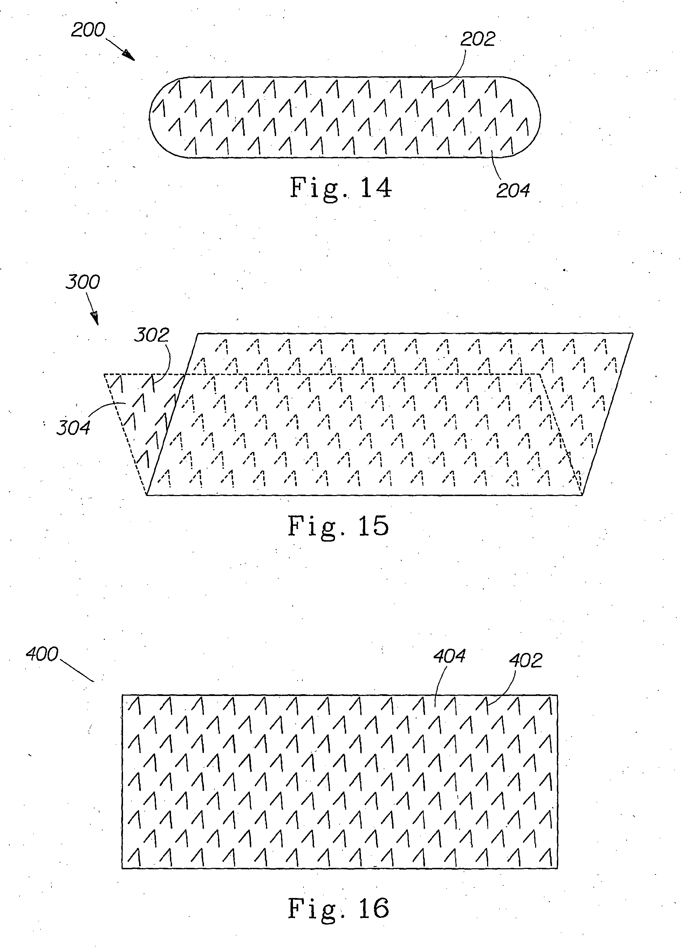

To minimize the contact area between the contact surface(s) of the article, the primary package and/or the liner, at least one of the contact surface(s) of the article, primary package and/or the liner, can be textured. As the amount of surface area of contact surfaces touching increases, the likelihood of the contact surfaces adhering to each other also increases. The texture be created with bumps, protrusions, dimples, depression, indentation and any combination of these, and the textures can be in a random or a pattern formation. The texture can be in the forms of stripes, spots, shapes such as squares, triangles, circles, etc and any combination thereof (as shown in Figs. 10-13). Fig. 10 depicts a denture adhesive article 70 comprising a stripe texture 72. Fig. 11 depicts a denture adhesive article 80 comprising a square texture 82. Fig. 12 depicts a denture adhesive article 90 comprising a triangle texture 92, and Fig. 13 depicts a denture adhesive article 100 comprising a honeycomb texture 102. Additionally, in Figs. 14-16 a raised texture is shown as protrusions 202 from the contact surface 204 in denture adhesive article 200, protrusions 302 from the contact surface 304 of the liner 300; and protrusions 402 from one of the contact surfaces 404 of the primary package 400. The texturing can be accomplished by any suitable method including: etching a pattern onto at least one contact surface; embossing; by shaping a pattern into at least one of the contact surface(s) of the article, the primary package and/or the liner during manufacture; and/or by printing a texture onto at least one contact surface of the article, the primary package and/or the liner etc. Additionally, when more than one of the contact surface(s) of the article, the primary package and/or the liner surfaces are textured, the texturing on contact surface of one of the article, primary package, and/or the liner can have a different texture than the other contact surface of the article, primary package and/or the liner. In one embodiment the article, the primary package and the liner all have one or more contact surface(s) that are textured. In another embodiment all of the contact surfaces of the liner and the article are textured. In yet another embodiment the contact surfaces of the liner, the article and primary package are textured.

In one embodiment, the denture adhesive delivery system includes a denture adhesive article which has textured contact surfaces, a liner which has textured contact surfaces and which is coated with silicone, and a primary package.

### PROCESS FOR PREPARATION OF THE ARTICLE

The articles utilized in accordance with the invention are formed by processes conventional in the arts, e.g. the film making industries such as casting, coating, calendaring, extrusion. In one embodiment the separate components of the article are melted and then blended in a mixing tank until a homogeneous mixture is achieved. Thereafter, the melted mixture may be cast to an acceptable thickness, on an appropriate substrate. Examples of such substrates include Mylar, continuous moving stainless steel belt (which may eventually entering a dryer section if needed), release paper and the like. The articles are then cooled. The articles may then be dried if needed, e.g. in a forced-air oven. The temperature of the drying air and length of drying time depend on the nature of the solvent utilized as is recognized in the art. Generally, the drying temperatures include a temperature between about 25°C and 140°C, in another embodiment from about 60° and 90° C for a duration of about 20 minutes to about 60 minutes, in another embodiment from about 30 to about 40 minutes. The article may then be cut into desired shapes with desired dimensions and then stacked and/or subsequently packaged.

In one embodiment, after processing, the article is then die-cut into desired shapes. These shapes may facilitate application of the article to the dentures.

In one embodiment in particular the present article is processed as follows: 1. melt the wax component; 2. mix the AVE/MA salt(s) with any other adhesive component; 3. add the adhesive mixture to the wax melt; 4. stir to made a homogeneous mixture; 5. pour the homogeneous mixture into mold or onto a suitable surface; 6. cool the mixture until it solidifies; 7. remove from substrate or mold or cut into the desired shape.

Another conventional film-making process known in the art is extrusion. This method is possible with films wherein the film forming ingredient comprises a variety of extrudable materials. The mechanical particulars of the extrusion process, e.g. the particular equipment utilized, the extruding force, the shape and temperature of the orifice and/or dies are considered to be within the skill of the art and can be varied in a known manner to achieve the physical characteristics of the articles described herein.

In one embodiment the thickness of the articles herein is generally between about 0.1 mm to about 2.5 mm, in another embodiment is from about 0.4 mm to about 1.5 mm thick, in another embodiment is from about 0.5 mm to about 1mm thick. The article may be thicker or thinner depending on the degree of cushioning desired by the user or wearer.

In one embodiment the articles herein may optionally be multiphase or have visually distinct phases. In another embodiment the articles herein may optionally have a release liner.

### ARTICLE USE

The present articles are generally applied to the denture prosthesis and thereafter the denture is secured to the oral cavity. In one embodiment the dentures are dried prior to application of the article. In one embodiment it is not necessary to wet the article and/or the denture prosthesis prior to applying it to the denture prosthesis in order to make the article stick to the denture prosthesis. The article may be applied to any suitable location on the prosthesis. In one embodiment the denture wearer generally wears the article from about 1 hour to about 3 days, in another embodiment from about 6 hours to about 24 hours. After usage the prosthesis is removed from the oral cavity, and any remaining article may be cleaned from the prosthesis, for example by gentle scrubbing with water and a brush.

### EXAMPLES

### Articles

The following examples further describe and demonstrate embodiments within the scope of the present invention. The examples are given solely for the purpose of illustration and are not to be construed as limitations of the present invention.

### EXAMPLE I

| | A | B | C | D | E | F | G | H | I |
|---|---|---|---|---|---|---|---|---|---|
| | Grams | Grams | Grams | Grams | Grams | Grams | Grams | Grams | Grams |
| Ca/Zn AVE/MA Salt | 33.00 | 33.00 | 33.00 | 53.00 | | 33.00 | 23.00 | 28.00 | 24.5 |
| CMC | 20.00 | 20.00 | 20.00 | | 53.00 | 25.00 | 30.00 | 15.00 | 28.5 |
| AVE/MA Acid S-97 | | | 1.0 | | | | | | |
| Microcrystalline | 46.92 | 47.00 | 47.00 | 47.00 | 47.00 | 42.00 | 47.00 | 45.52 | 46.92 |
| Flavors | | | 0.50 | | | | | 0.4 | |
| Saccharin | 0.08 | | 0.16 | | | | | 0.08 | 0.08 |
| Colorants | | | 0.10 | | | | | | |
| Silica | | | | | | | | 1.0 | |
| Corn Starch | | | | | | | | 10 | |

The Microcrystalline Wax W-835 (or Polyethylene AC 1702 or A-C 6702) is melted, and the other ingredients are blended with it. The mixture is then made into sheets by any suitable means such as extrusion or rolling into sheets of suitable thickness such as 0.50mm, 0.67mm, 0.73mm, or 1.0mm. The sheet is then cut into shapes suitable for application to dentures.

In Example I all or part of the Ca/Zn AVE/MA salt may be substituted with Mg/Zn/Na AVE/MA Salts and/or Ca/Na AVE/MA salts; all or part of the CMC may be substituted with caraggeenan, and/or suitable cellulose derivatives; all or part of the Microcystalline Wax W-835 may be substituted with Polyethylene A-C 1702 (available from Honeywell), and/or Polyethylene A-C 6702 (available from Honeywell); and/or, the amount of each ingredient may also be increased or decreased by up to about 50%. Each of the above examples may be blended with each other prior to making into articles; and/or used in conjunction with each other to form multilayer articles.

In one embodiment to measure bioerosion of the foregoing Examples, run a water source on top of the sample specimen for 30 minutes while the specimen sits atop a wire mesh. The water source is a laboratory faucet adjusted such that the temperature is 39 ± 1 °C and the flow rate is 16 ± 1 ml/sec. A funnel is used to focus the flow and help dampen the effect of small pressure and temperature fluctuations within the water lines. The wire mesh grid has square openings approximately 2,28 mm x 2,28 mm (0.09 inches x 0.09) inches) and is placed 63.5 mm (2.5 inches) below the tip of the funnel where it is clamped to a metal ring for support. Sample specimens weighing 0.025 g are placed on the mesh and images are taken at 0, 10 and 30 minutes to follow bio-erosion of the specimen. After 30 minutes the wire mesh containing the remainder of the specimen are removed and heated for 1 hour at 60°C under vacuum to remove all remaining water. After the heating period, final weights are taken to calculate weight loss due to bio-erosion. An average of 3 specimens per sample are used to calculate bio-erosion time and weight loss. In one embodiment the article is bioerodible if it does not leave behind visible residue, film, or sheet after about 30 minutes under these testing conditions. In another embodiment the article is bioerodible if it cannot be easily separated or peeled away manually in one or more large pieces after about 30 minutes under these testing conditions. In yet another embodiment the article is bioerodible if it leaves behind less than about 2, less than about 4, less than about 6, and/or less than about 8% by weight of residue (of the original weight of the article) after about 30 minutes under these testing conditions. In another embodiment the above bio-erosion test may also be conducted at various time-points up to 8 hours.

In one embodiment, the dry tack may be measured by the following procedure: a. Compress a 5mm diameter disc (0.67mm thick) sample of the article between a 1" diameter cylindrical probe (made from polymethylmethacrylate) and a flat sheet of polymethylmethacrylate with a 2000 gram-force for 2 seconds, b. Pull off the probe at 1mm/second and record peak force, c. Repeat procedure with no sample sandwiched between the two surfaces, and d. Calculate: Dry Tack in grams/square centimeter = (Peak Force with Sample - Peak Force without sample) / Cross sectional area of sample disc

### Non-Stick Coatings

The following examples illustrate various non-stick coatings. These coatings may be applied onto the primary package, liner, and/or article.
a. Silicone TT3796.000 from Nordenia Company
b. Release Pouch Wrap (RPW) film from Nordenia Company
c. SP5000.000 from Nordenia Company
d. Silicone
e. Heat-cured Silicone
f. UV-cured Silicone .
g. SL6625 Silicone from GE

Liners

The following examples illustrate various non-stick liners.
a. Parchment paper from Reynolds Company and Ahlstrom Company
b. Grillon paper from Reynolds Company and Ahlstrom Company
c. Unibake paper from Reynolds Company and Ahlstrom Company
d. Hydroformed films
e. Silicone rubber
f. Silicone foam

### TEST METHODS

The bioerosion of the inventive articles can be measured by the following method: run a water source on top of the sample specimen for about 30 minutes while the specimen sits atop a wire mesh. The water source is a laboratory faucet adjusted such that the temperature is 39 ± 1 °C and the flow rate is 16 ± 1 ml/sec. Use a funnel to focus the flow and help dampen the effect of small pressure and temperature fluctuations within the water lines. The wire mesh grid has square openings approximately 2.28x2.28 mm (0.09 inches x 0.09 inches) and is placed 63.5 mm (2.5 inches) below the tip of the funnel where it is clamped to a metal ring for support. Sample specimens weighing 0.025 g are placed on the mesh and images are taken at 0, 10 and 30 minutes to follow bio-erosion of the specimen. After 30 minutes the wire mesh containing the remainder of the specimen is removed and heated for 1 hour at 60 °C under vacuum to remove all remaining water. After the heating period, final weights are taken to calculate weight loss due to bio-erosion. An average of 3 specimens per sample are used to calculate bio-erosion time and weight loss. The article is bioerodible if it does not leave behind visible residue, film, or sheet after about 30 minutes under these testing conditions., and/or if it cannot be easily separated or peeled away manually in one or more large pieces after about 30 minutes under these testing conditions, and/or if it leaves behind less than about 2, less than about 4, less than about 6, and/or less than about 8% by weight of residue (of the original weight of the article) after about 30 minutes under these testing conditions. The above bio-erosion test may also be conducted at various time-points up to 8 hour.

The dry tack can be measured by the following method: 1. remove the article from the package material; 2. place the article on the palate-portion of a dry, acrylic upper-denture with the teeth facing downward; 3. apply pressure with fingers for about 3 to 10 seconds; 4. thereafter remove finger pressure; 5. then invert the denture with the teeth facing upward. In one embodiment the article demonstrates dry tack if: i. The article does not stick to fingers during steps 1-2, ii. leaves little or no residue on the fingers in steps 3-4, and iii. in step-5, the article does not fall off of the denture, once inverted, for at least about 10-30 seconds, or at least about 1 minute.

In another embodiment the article demonstrates dry tack if: i. The article does not stick to fingers during steps 1-4, and ii. in step-5, the article does not fall off of the denture, once inverted, for at least about 10-30 seconds, or at least about 1 minute.

In another embodiment the article demonstrates dry tack if in step-5, the article does not fall off of the denture, once inverted, for at least about 10-30 seconds, or at least about 1 minute.

The dry tack of the inventive articles can also be measured by the following procedure:
a. Compress a 5mm diameter disc (0.67mm thick) sample of the article between a 1" diameter cylindrical probe (made from polymethylmethacrylate) and a flat sheet of polymethylmethacrylate with a 2000 gram-force for 2 seconds,
b. Pull off the probe at 1mm/second and record peak force,
c. Repeat procedure with no sample sandwiched between the two surfaces, and
d. Calculate: Dry Tack in grams/square centimeter = (Peak Force with Sample - Peak Force without sample) / Cross sectional area of sample disc.

In one embodiment the above procedure is repeated with an applied force of 250 gram-force in step-a and the tack measured in steps b-d;

The article has dry tack if the tack measured with a 250 gram-force applied force is less than about 25, 50, 100, 200, or 500 grams/square-centimeter, and the tack measured with a 2000 gram-force applied force is greater than about 200, 500, 1000, 2000, 5000, 10000, or 25000 grams/square-centimeter, and any combination of these levels.

The modulus G' of the inventive article can be measured by the following procedure:
a. Load a sample disc of 8mm diameter and 0.67mm thickness onto a ARES rheometer using a parallel plate fixture with a compressive force of 500 grams. If the sample is flowable a sufficient amount of material is used to fill the 1mm gap on a 25 mm diameter parallel plate fixture.
b. Set strain to be 0.02%, c. Measure G' at a sweep of frequencies including 1 Hz.

The normalized dislodgement force and dislodgement force ratio of the inventive article can be measured by the following method:
Instrument: An Instron model 5544 is used. The load cell is calibrated according to manufacturer's specifications annually. The choice of load cell is determined by having the forces generated by the adhesive fall within the recommended operating range for the load cell. This is typically between 10% - 90% of full capacity.
Test Fixtures: The geometry of a cylindrical probe and a flat plate are used as the test fixtures. The probe is made from PMMA, 0.2 sq.cm to 10 sq.cm in surface area. For the base plate, the same PMMA material is used but in sheet form, ¼" thick. This is cut into 6" x 6" plates to be clamped onto the Instron.
Hydrating Liquid: Artificial saliva containing low levels of various salts is used to hydrate the adhesive.

### Artificial Saliva Composition

| Ingredient | | Amount per Liter |
|---|---|---|
| K₂HPO₄ | | 4.2 g |
| KH₂PO₄ | | 3.2 g |
| KOH | | 2 pellets |
| Mineral Stock Solution | | 5 ml |
| - KCl | 8 g per 100 ml of Stock Solution | |
| - NaCl | 8 g | |
| - Na2SO4 | 0.264 g | |
| - MgCl2.6H2O | 0.7687 | |
| | (or 0.36 g Anhydrous MgCl2) | |

Adhesive: 0.1 to 1.0 gram of adhesive is applied to the probe.
Hydration: The hydrating liquid (0.2 mL of artificial saliva to 2.0 ml) is pipetted onto the surface of the adhesive. The assembly is then permitted to hydrate for 20 minutes or more.
Test Method: Once the sample is hydrated, it is mounted onto the Instron and the test is carried out via computer control. The method is comprised of the following steps:
   (a) Compression to 750 to 7500 g of force
   (b) Hold at compression for 2 minutes
   (c) Reduce compressive force to 200 gf
   (d) Hold (1 minute)
   (e) Pull off at 1 mm/s
   (f) Record Peak Dislodgement Force
   (g) Calculate "Normalized Dislodgement Force" = (Peak Dislodgement Force) /(Surface Area of Probe); report in grams force per sq.cm
   (h) Repeat steps A-F for commercial Fixodent Original denture adhesive (available commercially manufactured by P&G), or for the following reference formula:
      Ca(47.5%)/Zn(17.5%) MVE/MA salt 33%, sodium carboxymethylcellulose 20%, mineral oil USP (65-75cst at 40C) 23.93%, petrolatum USP (consistency 17-20mm) 21.87%, colloidal silicon dioxide 1.14%, and Opatint OD1646 0.06%; suitable methods to make this reference formula are disclosed in US 5,073,604, Holeva K., and US 6,617, 374 Rajaiah J.
   (i) Calculate "Dislodgement Force Ratio" = (Peak Dislodgement Force of Prototype Adhesive)/(Peak Dislodgement Force of Fixodent Original)
      Data: Each sample is repeated a minimum of 3 times and the average value of the "Normalized Dislodgement Force" and "Dislodgement Force Ratio" are reported.

Specifically the normalized dislodgement force and dislodgement force ratio can be measured by using the following parameters in the procedure: 0.25 gram adhesive; 1 inch diameter probe; hydration time of 20 minutes; and compression force of 7500 grams.

The "normalized ooze amount" and "ooze ratio" of the inventive article can be measured by the following procedure:
a. Load initial sample weight of about 0.50 grams uniformly onto a 1 inch diameter cylindrical probe made from polymethylmethacrylate,
b. Bring probe to 1.2 mm of base plate, also made from polymethylmethacrylate,
c. Apply 750 gram force for 90 seconds,
d. At 90 seconds, trim and weigh material that has oozed out,
e. Calculate "Normalized Ooze Amount" = (Amount oozed out /Initial sample weight) x 100,
f. Repeat Steps a-e using commercial Fixodent Original a denture adhesive cream commercially manufactured by P&G, or with the following reference formula: Ca(47.5%)/Zn(17.5%) MVE/MA salt 33%, sodium carboxymethylcellulose 20%, mineral oil USP (65-75cst at 40C) 23.93%, petrolatum USP (consistency 17-20mm) 21.87%, colloidal silicon dioxide 1.14%, and Opatint OD1646 0.06%; suitable methods to make this reference formula are disclosed in US 5,073,604, Holeva K., and US 6,617, 374 Rajaiah J.,
g. Calculate "Ooze Ratio" = Normalized Ooze Amount of Prototype Adhesive / Normalized Ooze Amount of Fixodent Original,
h. Each sample is repeated a minimum of 3 times and the average value of the "Normalized Ooze Amount" and "Ooze Ratio" are reported.

## Claims

1. A denture adhesive article delivery system comprising:
(a) a denture adhesive article ready to use comprising a denture adhesive component and a water insoluble thermoplastic component selected from rubber ,elastomers, plastomers, natural wax, synthetic wax, polyvinyl chloride, nylon, fluoro-carbon, polyurethane prepolymer, polyethylene, polystyrene, polypropylene, cellulose resins, acrylic resins, petrolatum, polyvinyl acetate, and mixtures thereof; and
(b) a primary package to enclose and retain said denture adhesive article, the primary package having a non-stick coating for decreasing the sticking of a surface of said denture adhesive article to a surface of said primary package.

2. The denture adhesive article delivery system of Claim 1, wherein said denture adhesive article does not comprise a scrim.

3. The denture adhesive article delivery system of Claim 1, wherein said denture adhesive article has dry tack properties.

4. The denture adhesive article delivery system of Claim 1, wherein said coating is silicone.

## Patentansprüche

1. Zahnprothesen-Haftmittelartikel-Abgabesystem, umfassend:
(a) einen gebrauchsfertigen Zahnprothesen-Haftmittelartikel, umfassend einen Zahnprothesen-Haftmittelbestandteil und einen wasserunlöslichen thermoplastischen Bestandteil, ausgewählt aus Kautschuk, Elastomeren, Plastomeren, natürlichem Wachs, synthetischem Wachs, Polyvinylchlorid, Nylon, Fluorkohlenstoff, Polyurethanvorpolymer, Polyethylen, Polystyrol, Polypropylen, Celluloseharzen, Acrylharzen, Petrolatum, Polyvinylacetat und Mischungen davon, und
(b) eine primäre Verpackung zum Umschließen und Einbehalten des Zahnprothesen-Haftmittelartikels, wobei die primäre Verpackung eine nichtklebrige Beschichtung aufweist, um das Kleben einer Oberfläche des Zahnprothesen-Haftmittelartikels an einer Oberfläche der primären Verpackung zu verringern.

2. Zahnprothesen-Haftmittelartikel-Abgabesystem nach Anspruch 1, wobei der Zahnprothesen-Haftmittelartikel keinen Gitterstoff umfasst.

3. Zahnprothesen-Haftmittelartikel-Abgabesystem nach Anspruch 1, wobei der Zahnprothesen-Haftmittelartikel Trockenklebrigkeitseigenschaften aufweist.

4. Zahnprothesen-Haftmittelartikel-Abgabesystem nach Anspruch 1, wobei die Beschichtung Silikon ist.

## Revendications

1. Système d'alimentation d'article adhésif pour dentier comprenant :
(a) un article adhésif pour dentier prêt à utiliser comprenant un composant adhésif pour dentier et un composant thermoplastique insoluble dans l'eau, choisi parmi un caoutchouc, des élastomères, des plastomères, une cire naturelle, une cire synthétique, du chlorure de polyvinyle, du nylon, du fluorocarbone, un prépolymère de polyuréthane, du polyéthylène, du polystyrène, du polypropylène, des résines de cellulose, des résines acryliques, du pétrolatum, de l'acétate de polyvinyle, et leurs mélanges ; et
(b) un conditionnement primaire pour enfermer et retenir ledit article adhésif pour dentier, le conditionnement primaire ayant un revêtement antiadhésif pour diminuer l'adhérence d'une surface dudit article adhésif pour dentier à une surface dudit conditionnement primaire.

2. Système d'alimentation d'article adhésif pour dentier selon la revendication 1, dans lequel ledit article adhésif pour dentier ne comprend pas de mousseline.

3. Système d'alimentation d'article adhésif pour dentier selon la revendication 1, dans lequel ledit article adhésif pour dentier a des propriétés d'adhésivité à sec.

4. Système d'alimentation d'article adhésif pour dentier selon la revendication 1, dans lequel ledit revêtement est de la silicone.
